# EUROPÄISCHE PATENTSCHRIFT

(11) **EP 1 693 081 B1**
(45) Veröffentlichungstag und Bekanntmachung des Hinweises auf die Patenterteilung: **05.01.2011**
(21) Anmeldenummer: 06001511.2
(22) Anmeldetag: 25.01.2006
(51) Int. Cl.: A61M 16/10, A61M 16/04

(54) **Wärme- und Feuchtigkeitsaustauscher**
Heat and moisture exchanger
Echangeur de chaleur et d'humidité

(30) Priorität: 17.02.2005 DE 102005007234; 17.02.2005 DE 202005002536 U
(43) Veröffentlichungstag der Anmeldung: 23.08.2006
(73) Patentinhaber: Andreas Fahl Medizintechnik-Vertrieb GmbH, 51149 Köln (DE)
(72) Erfinder: Fahl, Andreas, 51107 Köln (DE)
(74) Vertreter: Maxton Langmaack & Partner

(56) Entgegenhaltungen:
- EP-A- 1 208 866
- EP-A2- 0 861 671
- DE-A1- 3 928 530
- DE-U1- 20 114 355
- DE-U1- 20 302 580
- DE-U1- 29 816 303
- US-A- 5 383 447
- US-A- 5 590 644
- US-A- 5 992 413
- US-A1- 2004 123 974

## Beschreibung

Die vorliegende Erfindung betrifft einen Feuchte- und Wärmeaustauscher insbesondere für laryngektomierte und/oder tracheotomierte Personen, gemäß dem Oberbegriff des Anspruchs 1.

Bei operativen Eingriffen im oberen Atemtrakt kann das Anlegen einer künstlichen Atem-öffnung (Tracheostoma) in der Luftröhre notwendig sein, damit unter Umgehung von Mundraum und Kehlkopf Luft direkt in die Lunge eingeatmet werden kann. Bei laryngektomierten, also kehlkopflosen Personen, bei welchen der Kehlkopf operativ entfernt wurde, muss das Tracheostoma dauerhaft offen gehalten und stabilisiert werden, wozu insbesondere Trachealkanülen, in der Regel aus einer Außen- und einer Innenkanüle bestehend, in das Tracheostoma eingesetzt werden. Aber auch der Einsatz so genannter "Tracheostoma-Button" ist hier möglich - insbesondere für Patienten, die ansonsten keine Trachealkanüle mehr benötigen.

Des Weiteren können in das Tracheostoma auch so genannte "Shunt-Ventile" eingesetzt werden, welche eine Wiederherstellung der Stimme ermöglichen. Schließlich können in das Tracheostoma auch Filtersysteme eingesetzt werden, sowohl bei Tracheotomierten als auch bei Laryngektomierten. Derartige Filtersysteme bestehen entweder aus einem Pflaster mit einem eingesetzten Filter oder aus einer üblicherweise selbstklebenden Basisplatte - in aller Regel aus Kunststoff gebildet -, in welche Filter unterschiedlichster Art einsetzbar sind.

Zu den Filtersystemen, welche für laryngo-tracheale Hilfsmittel wie Trachealkanülen, Shunt-Ventile, Tracheostoma-Button und Filtersysteme mit Pflaster oder Basisplatte eingesetzt werden, zählen die so genannten "Feuchte- und Wärmeaustauscher", auch "künstliche Nase" genannt. Diese dienen dazu, die bei laryngektomierten und auch tracheotomierten Patienten fehlenden Regulationsmechanismen zur Erwärmung und Befeuchtung der Atemluft nachzubilden und ein In-Kontakt-Bringen der Luftröhre mit verstärkt trockener, kalter und nicht gefilterter Luft zu vermeiden. Denn durch die hierdurch hervorgerufene Reizung tritt eine erhöhte Schleimproduktion mit der nachfolgenden Gefahr der Verborkung auf. Durch Feuchte- und Wärmeaustauscher wird nun die eingeatmete Luft angefeuchtet, erwärmt und gleichzeitig gefiltert. Hierdurch wird die vorstehend erwähnte Borkenbildung weitgehend vermieden. Dabei hilft das regelmäßige Tragen der künstlichen Nase insbesondere bei starker Sekretabsonderung, da durch das Anfeuchten der Schleimhäute in der Luftröhre die Sekretproduktion vermindert wird.

Feuchte- und Wärmeaustauscher für Laryngektomierte und auch Tracheotomierte weisen einen Filterkörper auf - in aller Regel aus Papier oder Schaumstoff gebildet - durch welchen die ein- und ausgeatmete Luft geleitet wird. Beim Ausatmen hält der Filterkörper Feuchtigkeit zurück, welche dann beim Einatmen in die Luftröhre transportiert wird. Aus dem Stand der Technik bekannte künstliche Nasen, auch "HME" genannt (humid and moisture exchanger), gibt es in einer großen Vielzahl unterschiedlicher Ausbildungen für unterschiedliche Adapter. Der Universaladapter gemäß DIN EN ISO 5356-1 weist dabei einen Durchmesser von 15 mm auf, es sind jedoch aus dem Stand der Technik auch weitere Adapter mit insbesondere größeren Durchmessern bekannt. Der Universaladapter wird insbesondere bei Trachealkanülen eingesetzt, wohingegen Tracheostoma-Button und Filtersysteme - insbesondere mit einer selbstklebenden Basisplatte oder mit einem Pflaster ausgebildet - einen größeren Adapter-Durchmesser aufweisen. Daher sind in aller Regel diejenigen für Trachealkanülen ausgebildeten künstlichen Nasen nicht einsetzbar in Verbindung mit Tracheostoma-Button oder den erwähnten Filtersystemen. Gelöst werden kann dieses Problem dadurch, dass Trachealkanülen mit einem Adaptermittel versehen werden, welcher sich von dem üblicherweise verwendeten Universaladapter auf einen größeren Durchmesser erweitert, so dass hier künstliche Nasen einsetzbar sind, welche beispielsweise für Tracheostoma-Button-Träger genutzt werden.

Durch den Einsatz eines wie vorstehend beschriebenen Adaptermittels vergrößert sich die Baugröße jedoch erheblich, was von den Nutzern als ausgesprochen nachteilig empfunden wird. Selbiges gilt auch für die aus dem Stand der Technik bekannten künstlichen Nasen, welche auf Universaladapter aufgesetzt werden können. Denn diese weisen oftmals eine große Bauhöhe auf, wie beispielsweise die in der DE 201 14 355 U1 offenbarte.

Ein Feuchte- und Wärmeaustauscher gemäß dem Oberbegriff des Anspruchs 1 ist aus der EP 0 861 671 A2 bekannt. Die EP 0 861 671 A2 betrifft eine Gasbehandlungsvorrichtung und offenbart einen Feuchte- und Wärmeaustauscher mit einem Filterteil, umfassend einen Filterkörper und ein Filtergehäuse, und mit einem rohrförmig ausgebildeten Ansatzteil, wobei das Ansatzteil doppelwandig mit einer Außenwand und einer Innenwand ausgebildet ist. Der Filterkörper ist durch eine äußere Randfläche der Bodenplatte gehalten.

Die EP 1 208 866 A2 offenbart eine selbstbefeuchtende Tracheostoma-Vorrichtung umfassend ein trogartiges Gehäuse mit einem in diesem angeordneten Austauschelement, wobei dieses Austauschelement eine Vielzahl von laminaren Elementen aus Zellulose oder zelluloseähnlichem Material enthält.

Die DE 39 28 530 A1 offenbart einen Wärme- und Feuchtigkeitsaustauscher, der zwischen einem Y-Stück oder Nichtrückatemventil eines Beatmungssystems und einem Patienten angeordnet ist, bestehend aus einem Gehäuse mit einem zwei Schichten aufweisenden zylindrischen Wärme- und Feuchtigkeitsaustauschelements sowie einem im Gehäuseboden angeordneten Patientenanschluss und einem Im Gehäusedeckel angeordneten Geräteanschluss. Die patientennahe Schicht des Wärme- und Feuchtigkeitsaustauschelementes ist ein Drahtgewebe und die zweite Schicht ein Filtervlies. Patientennah ist im Abstand zum Patientenanschluss und zur Gehäusewand eine im Innenraum des Wärme- und Feuchtigkeitsaustauschelementes verschließbarer Einsatz angeordnet. Ein zwischen dem Gehäuse und dem Wärme- und Feuchtigkeitsaustauschelement gebildeter ringförmiger Luftraum ist mit dem Patientenanschluss verbunden.

Die US 5 590 644 offenbart einen in einem Gehäuse angeordneten scheibenförmigen Filter und Feuchtigkeits- und Wärmeaustauschelement mit Anschlussmitteln für flexible Atemwegsschläuche.

Die US 5 992 413 offenbart eine Vorrichtung zur Erwärmung und Befeuchtung von Atemwegsgasen, umfassend ein Gehäuse, ein gasdurchlässiges Element und weiteres Material. Das Gehäuse weist dabei Ansatzteile zum Anschluss an Schläuche oder sonstige Vorrichtungen auf, wobei ausgehend von dem Ansatzteil und der Innenwandung des Gehäuses Rippen hervorstehen, welche einen Filterkörper halten.

Es ist daher Aufgabe der vorliegenden Erfindung, einen insbesondere klein bauenden und für verschiedene Adaptergrößen geeigneten Feuchte- und Wärmeaustauscher zur Verfügung zu stellen.

Diese Aufgabe wird erfindungsgemäß gelöst durch einen Feuchte- und Wärmeaustauscher, insbesondere für Laryngektomierte und/oder Tracheotomierten, gemäß dem Anspruch 1. "Gehalten" im Sinne der vorliegenden Erfindung bedeutet, dass der Halt des Filterkörpers schon allein durch eine Auflage desselben auf einer Teilfläche der distalen Stirnfläche der Innenwand des Ansatzteils ermöglicht ist. Durch den Halt soll ein Durchrutschen des Filterkörpers durch das Ansatzteil bei der Einatmung sicher vermieden werden. Aber auch ein Halt durch Klebungen und sonstige Hilfsmittel fällt im Sinne der Erfindung unter den Begriff "gehalten".

Der erfindungsgemäße Feuchte- und Wärmeaustauscher weist den großen Vorteil auf, dass durch die Ausbildung eines rohrförmigen Ansatzteils eine äußere Umfangsfläche sowie eine innere Umfangsfläche zur Verfügung gestellt wird, welche einem Verbindungselement eines Adapterteils eines laryngo-trachealen Hilfsmittels angeformt ist. Dabei kann insbesondere die innere Umfangsfläche derart ausgebildet sein, dass ein Universaladapter gemäß DIN EN ISO 5356-1 mit dem erfindungsgemäßen Feuchte- und Wärmeaustauscher sicher verbindbar ist, wohingegen die Außnumfangsfläche Verbindungselementen mit einem größeren Durchmesser angeformt ist, beispielsweise einem Tracheostoma-Button. Damit ist der erfindungsgemäße Feuchte- und Wärmeaustauscher geeignet, für zwei unterschiedliche aus dem Stand der Technik bekannte Adaptergrößen angepasst und somit auch auf unterschiedlichen laryngo-trachealen Hilfsmitteln angeordnet, bzw. mit diesen verbunden zu werden.

Des Weiteren ist der erfindungsgemäße Feuchte- und Wärmeaustauscher sehr klein bauend ausgebildet, da durch die zumindest teilweise Auflage des Filterkörpers auf einer Teilfläche der distalen Stirnfläche der Innenwand des rohrförmigen Ansatzteils das Filterteil und insbesondere das von diesem umfasste Filtergehäuse klein bauend ausgebildet sein kann, so dass der erfindungsgemäße Feuchte- und Wärmeaustauscher eine Bauhöhe aufweist, welche mit üblicherweise aus dem Stand der Technik verwendeten und beispielsweise in Filtersystemen mit einer selbstklebenden Basisplatte einsetzbaren vergleichbar ist. Zudem kann der erfindungsgemäße Feuchte- und Wärmeaustauscher in seinem rohrförmig ausgebildeten Ansatzteil unterschiedliche Querschnitte aufweisen, so kann dieser beispielsweise oval oder ellipsenförmig ausgebildet sein. Besonders bevorzugt ist dabei jedoch ein ringförmiger Querschnitt. Es kann auch vorgesehen sein, dass die äußere Umfangsfläche einen ovalen oder ellipsenförmigen Querschnitt und die innere Umfangsfläche einen kreisförmigen Querschnitt aufweist und umgekehrt. Dabei kann das rohrförmige Ansatzteil bevorzugt einstückig mit dem Filterteil verbunden sein - es kann jedoch auch auf jede andere bekannte Weise mit diesem verbindbar sein, soweit hier eine luftdichte Verbindung hergestellt wird. Die Verbindung sollte des Weiteren eine ausreichende Festigkeit aufweisen, so dass eine sichere Entnahme des Feuchte- und Wärmeaustauschers aus dem laryngo-trachealen Hilfsmittel ermöglicht ist.

Das rohrförmige Ansatzteil ist doppelwandig mit einer Außenwand und einer Innenwand ausgebildet, wobei das distale Ende der Innenwand frei stehend ausgebildet ist. Zwischen Außen- und Innenwand ist dabei bevorzugt ein Hohlraum, weiter bevorzugt ein insbesondere ringförmiger Hohlraum, ausgebildet. Die Innenwand kann mit der Außenwand verbunden sein beispielsweise durch Stege o. ä. In einer bevorzugten Ausführungsform der vorliegenden Erfindung wird jedoch eine hinreichende Stabilisierung der Innenwand durch die Ausbildung einer geschlossenen Stirnfläche am proximalen Ende des Ansatzteils erreicht, wodurch die proximalen Stirnflächen der Außen- und der Innenwand miteinander verbunden sind. "Proximales Ende" im Sinne der vorliegenden Erfindung bezeichnet das der Körpermitte des Nutzers des erfindungsgemäßen Feuchte- und Wärmeaustauschers zugewandte Ende, wohingegen im Sinne der vorliegenden Erfindung mit "distalem Ende" bezeichnet ist das der Körpermitte des Nutzers entferntere Ende des Ansatzteils oder auch des Filterteils.

In einer bevorzugten Ausführungsform der vorliegenden Erfindung weist die distale Stirnfläche der Innenwand des Ansatzteils mindestens ein Fixiermittel, bevorzugt mindestens zwei, weiter bevorzugt mindestens drei Fixiermittel, für den Filterkörper auf. Durch die Fixiermittel, welche beispielsweise pin- oder spitzenartig ausgebildet sein können, wird zusätzlich durch den durch die distale Stirnfläche der Innenwand des rohrförmigen Ansatzteils vermittelten Halt des Filterkörpers diesem eine Fixierung gegeben, so dass dieser in seiner Bewegung im erfindungsgemäßen Feuchte- und Wärmeaustauscher gehindert ist. Dabei dringen die Fixiermittel bevorzugt in den Filterkörper, welcher aus Papier oder Schaumstoff gebildet sein kann, ein, so dass insbesondere eine Drehung des Filterkörpers im Filtergehäuse verhindert ist.

Vorzugsweise ist der Innendurchmesser des Ansatzteils des erfindungsgemäßen Feuchte- und Wärmeaustauschers kleiner als der Durchmesser des Filterkörpers, und der Außendurchmesser ist gleich oder größer als der Durchmesser des Filterkörpers ausgebildet. Hierdurch wird eine kleinbauende und auch in ihrem Außenumfang kleine Baugröße des erfindungsgemäßen Feuchte- und Wärmeaustauschers erzielt. Selbstverständlich ist es jedoch auch möglich, dass bei notwendigem Einsatz eines größeren Volumens des Filterkörpers das Filtergehäuse einen größeren Querschnitt zur Aufnahme eines entsprechend größeren Filterkörpers aufweisen kann, wozu zwischen Ansatzteil und Filterkörper ein Weitungsteil angeordnet sein kann. Dieses Weitungsteil kann dabei insbesondere kegelstumpfförmig ausgebildet sein. Bevorzug ist jedoch das Ansatzteil unmittelbar benachbart dem Filterteil angeordnet. Bei einer derartigen Ausbildung kann der Einsatz eines Filterkörpers mit einem größeren Querschnitt dadurch erzielt werden, dass ein entsprechend vergrößerter Haltebund, welcher zum Filtergehäuse des Filterteils gehört, vorgesehen wird, welcher unmittelbar mit dem Ansatzteil verbunden ist. Dieser Haltebund ist dabei insbesondere einstückig mit dem Ansatzteil verbunden.

In einer bevorzugten Ausführungsform weist das Ansatzteil und/oder das Filterteil einen ringförmigen Querschnitt auf. Hierdurch ist eine einfache Anformung insbesondere an den Universaladapter gemäß DIN EN ISO 5356-1 ermöglicht. Des Weiteren können derartige erfindungsgemäße Feuchte- und Wärmeaustauscher relativ einfach durch entsprechend ausgebildete Spritzformen erzeugt werden. Zudem ist es bei einem ringförmigen Querschnitt auch leicht möglich, das Filterteil mit einem entsprechend größeren ringförmigen Querschnitt bei einem notwendigen größeren Filtervolumen zur Verfügung zu stellen.

Vorteilhafterweise ist an der Außenumfangsfläche des Ansatzteils an dessen dem Filterteil abgewandten Ende, d.h. dessen proximalem Ende, ein umlaufender Wulst angeordnet. Durch diesen umlaufenden Wulst kann eine Rastung und insbesondere ein sicherer Halt bei Einsatz in laryngo-trachealen Hilfsmitteln mit entsprechenden Adaptergrößen erfolgen.

In einer vorteilhaften Ausführungsform der vorliegenden Erfindung ist die Baulänge der Innenwand kleiner als die Baulänge der Außenwand ausgebildet. Dabei ist die Baulänge des die Innenwand bildenden Rohrstutzens mindestens 5, bevorzugt mindestens 10, weiter bevorzugt mindestens 15% geringer als diejenige des die Außenwand bildenden Rohrstutzens des Ansatzteils. Dabei sollte jedoch die Länge des die Innenwand bildenden Rohrstutzens bevorzugt mindestens 50% derjenigen des die Außenwand bildenden Rohrstutzens des Ansatzteils betragen, um eine hinreichend sichere Verbindung insbesondere mit einem Universaladapter gemäß DIN EN ISO 5356-1 zu ermöglichen. Dabei können an der Innenumfangsfläche der Innenwand - ähnlich wie an der Außenumfangsfläche der Außenwand - noch bevorzugt vollständig umlaufende Wulste oder Nuten, aber auch sonstige Einkerbungen oder Vorsprünge angeordnet sein, um eine hinreichend feste, gleichzeitig aber auch wieder lösbare Verbindung sicherzustellen.

Vorzugsweise weist das Filtergehäuse des Filterteils des erfindungsgemäßen Feuchte-und Wärmetaustauschers mindestens eine seitliche Ausnehmung, bevorzugt mindestens zwei, weiter bevorzugt mindestens drei, noch weiter bevorzugt genau vier Ausnehmungen auf. Durch die Vorsehung seitlicher Ausnehmungen wird der Weg der ein- und ausgeatmeten Luft durch den erfindungsgemäßen Feuchte- und Wärmeaustauscher je nach Dimensionierung des Filterkörpers verlängert. Zudem kann insbesondere bei einer Ausbildung des Filtergehäuses dahingehend, dass dieses mit sehr großen seitlichen Ausnehmungen versehen ist und daher einen nur noch skelettartigen Aufbau aufweist, eine große Fläche des Filterkörpers der ein- und ausströmenden Luft verfügbar gemacht werden.

Bevorzugt weist das Filtergehäuse einen ersten Haltebund auf, der beabstandet vom Ansatzteil durch mindestens zwei Stege gehalten ist. Durch die mindestens zwei, bevorzugt drei, weiter bevorzugt genau vier Stege ist ein skelettartiger, gleichwohl hinreichend stabiler Aufbau und Ausbildung des Filtergehäuses des Filterteils ermöglicht. Das Filterteil weist dabei bevorzugt an seinem proximalen Ende einen weiteren mit dem Ansatzteil verbundenen Haltebund auf, welcher, wie bereits vorstehend beschrieben, in seinem Außenumfang je nach eingesetztem Filterkörper auch vergrößert ausgebildet sein kann. Entsprechend ist dann auch der erste Haltebund des Filtergehäuses mit einem größeren Außenumfang versehen. Das Filterteil umfasst dabei ein Filtergehäuse mit dem ersten und dem weiteren Haltebund und die die beiden Haltebunde verbindenden Stege, sowie einen Filterkörper. Auch wenn die rohrstutzenförmige Innenwand eine geringere Bauhöhe aufweist als die rohrstutzenförmige Außenwand und daher der Filterkörper des Filterteils im Bereich des Ansatzteils auf der distalen Stirnfläche der Innenwand zumindest teilweise zu liegen kommt, gehört im Sinne der vorliegenden Erfindung der Filterkörper nicht zum Ansatzteil.

Vorteilhafterweise weist der Steg, angeordnet zwischen erstem Haltebund und weiterem Haltebund, ein Stabilisierungselement auf. Das Stabilisierungselement soll dabei der Festigkeitserhöhung bei Ausbildung eines im Wesentlichen skelettartig aufgebauten Filtergehäuses dienen. Vorteilhafterweise ist dabei das Stabilisierungselement, ausgehend vom ersten Haltebund zum weiteren Haltebund hin in Umfangsrichtung gesehen sich verbreiternd ausgebildet. Dabei ist das Stabilisierungselement bevorzugt im Wesentlichen dreieckig in Umfangsrichtung gesehen ausgebildet. Entsprechend der Größe des Stabilisierungselements ist der weitere Haltebund in seinem Außenumfang vergrößert ausgebildet, so dass dieser eine Art Grundplatte bildet, auf welcher Steg und Stabilisierungselement angeordnet sind. Steg und Stabilisierungselement sind dabei bevorzugt wiederum einstückig ausgebildet. Bei einer derartigen Vergrößerung des weiteren Haltebundes kann vorgesehen sein, dass dieser eine umlaufende Kerbung zur Bildung eines Rücksprungs auf seiner proximalen Seite aufweist, was die Festigkeit und Greifbarkeit des erfindungsgemäßen Feuchte- und Wärmeaustauschers weiter erhöht.

In einer weiter bevorzugten Ausführungsform des erfindungsgemäßen Feuchte- und Wärmeaustauschers weist der erste Haltebund an seiner Außenumfangsfläche einen umlaufenden Haltewulst auf. Mittels dieses umlaufenden Haltewulstes ist vorteilhafterweise ein Sicherheitsventil, welches vom Feuchte- und Wärmeaustauscher umfasst ist, gehalten. Das Sicherheitsventil, auch "Überdruckventil" genannt, ermöglicht ein Abhusten von Sekreten, ohne dass das Entfernen des Feuchte- und Wärmeaustauschers aus dem Adapter des laryngo-trachealen Hilfsmittels notwendig ist. Das Sicherheitsventil ist dabei aus einem weichen Kunststoff oder aber Silikon gebildet und weist bevorzugt eine sternförmige Mittelstanzung auf.

In einer weiter vorteilhaften Ausbildung des erfindungsgemäßen Feuchte- und Wärmeaustauschers weist der Filterkörper des Filterteils eine zentrale Bohrung auf. In Kombination mit dem Sicherheitsventil ist dadurch ein Abhusten von Sekreten ohne Entfernen des erfindungsgemäßen Feuchte- und Wärmeaustauschers möglich. Der erfindungsgemäße Feuchte- und Wärmeaustauscher kann des Weiteren insbesondere am Filterteil, aber auch am Ansatzteil angeordnet einen Stutzen zum Anschluss eines Sauerstoffgeräts aufweisen. Insbesondere bei der Ausbildung des Ansatzteils des erfindungsgemäßen Feuchte- und Wärmeaustauschers, bei welchem die Baulänge der rohrstutzenförmigen Innenwand kleiner als diejenige der rohrstutzenförmigen Außenwand ist, ist der Stutzen angeordnet im Bereich des dann in das Ansatzteil hineinragenden Filterkörpers des Filterteils. Hierdurch kann die durch ein Sauerstoffgerät zugeführte Luft zunächst durch den Filterkörper geführt und hierdurch erwärmt, angefeuchtet und gefiltert werden.

Bei der Verwendung eines erfindungsgemäßen Feuchte- und Wärmeaustauschers als künstliche Nase zum Einsatz in Trachealkanülen, Tracheostoma-Button, Shunt-Ventilen und/oder Filtersystemen mit selbstklebender Basisplatte oder mit Pflaster ist es möglich, dass sich der Nutzer des erfindungsgemäßen Feuchte- und Wärmeaustauschers beispielsweise bei Umstellung von einer eingesetzten Trachealkanüle auf einen eingesetzten Tracheostoma-Button im Hinblick auf die zu verwendende künstliche Nase nicht an andere Ausführungsformen gewöhnen und umstellen oder ein zusätzliches Adaptermittel einsetzen muss, um die bisher von diesem verwendete künstliche Nase weiterzuverwenden.

Diese und weitere Vorteile der vorliegenden Erfindung werden anhand der folgenden Figuren näher erläutert.

Es zeigen:
- Fig. 1:: eine perspektivische Ansicht des erfindungsgemäßen Feuchte- und Wär- meaustauschers;
- Fig. 2:: eine Seitenansicht des Feuchte- und Wärmeaustauschers gemäß Fig. 1;
- Fig. 3:: eine perspektivische Ansicht von schräg unten einer weiteren Ausführungs- form Feuchte- und Wärmeaustauschers;
- Fig. 4:: eine Unteransicht des Feuchte- und Wärmeaustauschers gemäß Fig. 1;
- Fig. 5: das Sicherheitsventil gemäß dem Feuchte- und Wärmeaustauscher gemäß Fig. 1 in einer Einzelansicht;
- Fig. 6:: den Filterkörper des Feuchte- und Wärmeaustauschers gemäß Fig. 1 in einer Einzelansicht; und
- Fig. 7:: das Filtergehäuse und das Ansatzteil des Feuchte- und Wärmeaustau- schers gemäß Fig. 1 in einer perspektivischen Ansicht.

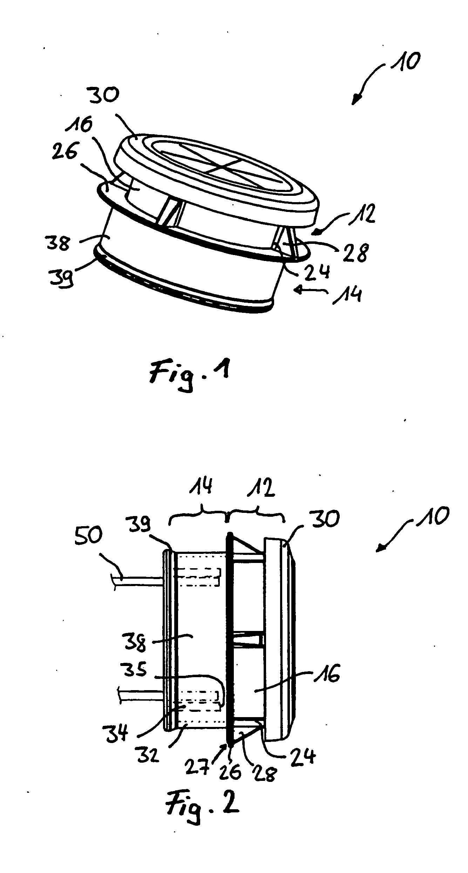

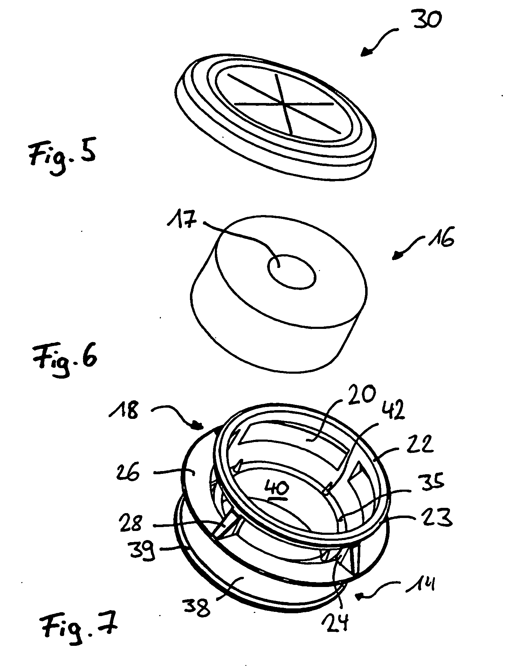

Fig. 1 zeigt einen erfindungsgemäßen, insgesamt mit dem Bezugszeichen 10 bezeichneten Feuchte- und Wärmeaustauscher. Dieser weist ein Filterteil 12 und ein Ansatzteil 14 sowie ein Sicherheitsventil 30 auf, welches auf dem Filterteil 12 aufgesetzt ist. Des Weiteren weist der Feuchte- und Wärmeaustauscher 10 einen Filterkörper 16 auf. Der Filterkörper 16 ist im Detail in Fig. 6 gezeigt. Der Filterkörper 16 ist ringförmig ausgebildet und weist in der Mitte eine Bohrung 17 auf. Der Filterkörper 16 kann dabei aus Papier oder Schaumstoff, beispielsweise einem Polyether-Schaumstoff, oder sonstigen für medizinische Anwendungen geeigneten Schaumstoffen, auch beschichtet, ausgebildet sein. Das Sicherheitsventil 30 ist in einer Einzeldarstellung in Fig. 5 gezeigt und weist eine sternförmige Stanzung auf. Es ist bevorzugt aus Silikon gebildet und ermöglicht dem Träger ein Abhusten von Sekreten ohne ein Entfernen des Feuchte- und Wärmeaustauschers 10 aus einem laryngo-trachealen Hilfsmittel, wobei die Sekrete dann durch die Bohrung 17 des Filterkörpers 16 nach außen geführt werden.

Der Feuchte- und Wärmeaustauscher 10 weist gemäß Fig. 1 in seinem Filterteil 12 einen Haltebund 26 auf, auf welchem Stege 24 angeordnet sind, welche weiterhin in etwa dreieckig ausgebildete Stabilisierungselemente 28 aufweisen. Dabei ist der Haltebund 26 ringplattenförmig ausgebildet, so dass die sich auf das proximale Ende des Filterteils 12 hin verbreiternden Stabilisierungselemente 28 mit ihrer kurzen Seite vollständig auf dem Haltebund 26 aufliegen.

Das Ansatzteil 14 ist ebenso wie das Filterteil 12 ringförmig ausgebildet und weist eine Außenumfangsfläche 38 und einen umlaufenden Wulst 39 auf, welcher die Verbindung zum Verbindungselement eines Adapters eines laryngo-trachealen Hilfsmittels weiter verbessert.

Fig. 2 zeigt den Feuchte- und Wärmeaustauscher 10 gemäß Fig. 1 in einer Seitenansicht. Dabei ist schematisch angedeutet der Aufsatz des Feuchte- und Wärmeaustauschers 10 auf einem Verbindungselement 50 eines hier nicht näher gezeigten Adapters eines laryngo-trachealen Hilfsmittels, beispielsweise eines Universaladapters gemäß DIN EN ISO 5356-1 einer Trachealkanüle. Der Feuchte- und Wärmeaustauscher 10 weist in seinem Ansatzteil 14 eine Außenwand 32, welche mit ihrem distalen Ende in dem Haltebund 26 endet, und eine rohrstutzenförmig ausgebildete Innenwand 34 (schematisch angedeutet) auf, welche eine distale Stirnfläche 35 aufweist. Das Verbindungselement 50 ist mit seiner Außenumfangsfläche der Innenumfangsfläche der Innenwand 34 angeformt, so dass ein sicherer Halt des Feuchte- und Wärmeaustauschers 10 auf dem Universaladapter des Verbindungselements 50 ermöglicht ist. Das Ansatzteil 14 umfasst somit gemäß Fig. 2 die Außenwand 32, die rohrstutzenförmig ausgebildete Innenwand 34 sowie den umlaufenden Wulst 39. Demgegenüber umfasst das Filterteil 12 den Haltebund 26, welcher eine umlaufende Kerbe 27 zur Ausbildung eines Rücksprungs aufweist, wodurch eine Stabilisierung des Feuchte- und Wärmeaustauschers 10 erhalten wird. Des Weiteren weist das Filterteil 12 die aus Fig. 2 besonders gut ersichtlichen Stege 24 und Stabilisierungselemente 26 auf.

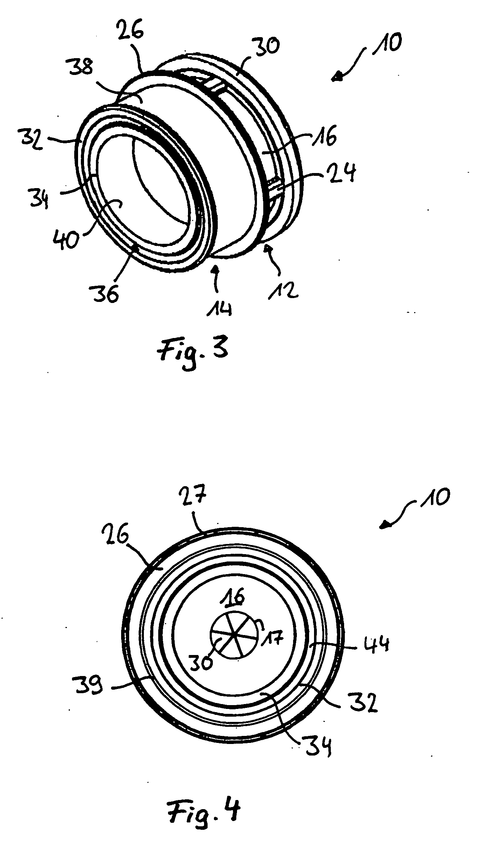

Fig. 3 zeigt in einer perspektivischen Ansicht von schräg unten einen Feuchte- und Wärmeaustauscher 10, der im Unterschied zu demjenigen in Fig. 1 gezeigten keine Stabilisierungselemente 28 aufweist, so dass hier eine Ausbildung mit Stegen 24 und einem entsprechend etwas kleiner dimensionierten Haltebund 26 verdeutlicht ist. In Fig. 3 ist besonders gut die doppelwandige Ausbildung mit einer rohrstutzenförmigen Außenwand 32 und einer rohrstutzenförmigen Innenwand 34 ersichtlich mit der der Außenwand 32 zugeordneten Außenumfangsfläche 38 und der der Innenwand 34 zugeordneten Innenumfangsfläche 40. Dabei weist die Stirnfläche des Ansatzteils 14 eine geschlossene Ausbildung auf, so dass der zwischen der rohrstutzenförmigen Außenwand 32 und der rohrstutzenförmigen Innenwand 34 vorhandene Raum durch eine Verbindungsfläche geschlossen ist, so dass eine proximale Stirnfläche 36 geschaffen ist. Hierdurch ist eine ausreichende Stabilisierung der rohrstutzenförmigen Innenwand 34 im Feuchte- und Wärmeaustauscher 10 erreicht.

Fig. 4 zeigt eine Unteransicht des in Fig. 1 gezeigten Feuchte- und Wärmeaustauschers 10 und verdeutlicht insbesondere den Aufbau der Außenwand 32 und der Innenwand 34 mit dem zwischen diesen gebildeten Hohlraum 44. Des Weiteren ist im Filterkörper 16 deutlich die Bohrung 17 zu erkennen. Die umlaufende Kerbe 26 des Haltebundes 26 des Filterteils 12 ist ebenso gut zu erkennen wie die Ausbildung des auf der Außenumfangsfläche 38 umlaufenden Wulstes 39 der Außenwand 32.

Fig. 7 zeigt in einer perspektivischen Ansicht das Ansatzteil 14 mit dem Filtergehäuse 18 ohne Sicherheitsventil 30 und Filterkörper 16. Das Filtergehäuse 18 umfasst dabei den Haltebund 26 und den ersten Haltebund 22, an dessen Umfangsfläche ein Haltewulst 23 zur Vermittlung eines sicheren Halts des Sicherungsventils 30 ausgebildet ist. Insgesamt vier Stege 24 und vier Stabilisierungselemente 28, in etwa dreieckig ausgebildet, weist das Filtergehäuse 18 des Weiteren auf. Gut ist Fig. 7 zu entnehmen die distale Stirnfläche 35 der Innenwand 34 sowie die auf dieser angeordneten insgesamt vier pin- bzw. spitzenartig ausgebildeten Fixiermittel 42, durch welche der Filterkörper 16 in seiner Bewegung im Filtergehäuse des Feuchte- und Wärmeaustauschers 10 gehindert ist. Des Weiteren ist die Innenumfangsfläche 40 der Innenwand 34 gut zu erkennen. Das Filtergehäuse 18 weist insgesamt vier Ausnehmungen 20 auf und ist als in etwa skelettartig aufgebaut anzusehen. Eine ausreichende Festigkeit wird durch die Vergrößerung des Haltebundes 26 und der Stabilisierungselemente 28 erzielt.

Durch die vorliegende Erfindung wird somit ein Feuchte- und Wärmeaustauscher zur Verfügung gestellt, welcher einerseits kleinbauend ausgebildet ist, andererseits durch seine Anpassbarkeit an zwei Adaptergrößen, wobei eine insbesondere dem Universaladapter gemäß DIN EN ISO 5356-1 entspricht, vielfältige Einsatzmöglichkeiten bei laryngo-trachealen Hilfsgeräten wie Trachealkanülen, Tracheostoma-Button, Shunt-Ventilen oder Filtersystemen mit selbstklebender Basisplatte oder mit Pflaster bietet.

## Patentansprüche

1. Feuchte- und Wärmeaustauscher (10), insbesondere für Laryngektomierte und/oder Tracheotomierte, mit
- einem Filterteil (12), umfassend einen Filterkörper (16) und ein Filtergehäuse (18); und
- einem rohrförmig ausgebildeten Ansatzteil (14):
wobei
- das Ansatzteil (14) doppelwandig mit einer Außenwand (32) und einer Innenwand (34) ausgebildet ist;
**dadurch gekennzeichnet, dass**:
- das distale Ende der Innenwand (34) frei stehend ausgebildet ist und eine distale Stirnfläche (35) trägt; und
- der Filterkörper (16) zumindest teilweise durch eine Teilfläche der distalen Stirnfläche (35) gehalten ist.

2. Feuchte- und Wärmeaustauscher (10) gemäß Anspruch 1 , **dadurch gekennzeichnet, dass** die distale Stirnfläche (35) der Innenwand (34) des Ansatzteils (14) mindestens ein Fixiermittel (42) für den Filterkörper (16) aufweist.

3. Feuchte- und Wärmeaustauscher (10) gemäß einem der vorhergehenden Ansprüche, **dadurch gekennzeichnet, dass** das Ansatzteil (14) unmittelbar benachbart dem Filterteil (12) angeordnet ist.

4. Feuchte- und Wärmeaustauscher (10) gemäß einem der vorhergehenden Ansprüche, **dadurch gekennzeichnet, dass** das Ansatzteil (14) und/oder das Filterteil (12) einen ringförmigen Querschnitt aufweisen.

5. Feuchte- und Wärmeaustauscher (10) gemäß einem der vorhergehenden Ansprüche, **dadurch gekennzeichnet, dass** auf einer Außenumfangsfläche (38) des Ansatzteils (14) an dessen dem Filterteil (14) abgewandten Ende ein umlaufender Wulst (39) angeordnet ist.

6. Feuchte- und Wärmeaustauscher (10) gemäß einem der Ansprüche 2 bis 5, **dadurch gekennzeichnet, dass** die Baulänge der Innenwand (34) kleiner ist als die Baulänge der Außenwand (32).

7. Feuchte- und Wärmeaustauscher (10) gemäß einem der Ansprüche 2 bis 6, **dadurch gekennzeichnet, dass** zwischen der Außenwand (32) und der Innenwand (34) des Ansatzteils 14 ein ringförmiger Hohlraum (44) gebildet ist.

8. Feuchte- und Wärmeaustauscher (10) gemäß einem der vorhergehenden Ansprüche, **dadurch gekennzeichnet, dass** das Filtergehäuse (12) mindestens eine seitliche Ausnehmung (20) aufweist.

9. Feuchte- und Wärmeaustauscher (10) gemäß einem der vorhergehenden Ansprüche, **dadurch gekennzeichnet, dass** das Filtergehäuse (12) einen ersten Haltebund (22) aufweist, der beabstandet vom Ansatzteil (14) durch mindestens zwei Stege (24) gehalten ist.

10. Feuchte- und Wärmeaustauscher (10) gemäß Anspruch 9, **dadurch gekennzeichnet, dass** das Filterteil (12) an seinem proximalen Ende einen weiteren, mit dem Ansatzteil (14) verbundenen Haltebund (26) aufweist.

11. Feuchte- und Wärmeaustauscher (10) gemäß Anspruch 10, **dadurch gekennzeichnet, dass** der Außenumfang des weiteren Haltebundes (26) größer ist als derjenige des ersten Haltebundes (22).

12. Feuchte- und Wärmeaustauscher (10) gemäß einem der Ansprüche 9 bis 11, **dadurch gekennzeichnet, dass** der Steg (24) mindestens ein Stabilisierungselement (28) aufweist.

13. Feuchte- und Wärmeaustauscher (10) gemäß Anspruch 12, **dadurch gekennzeichnet, dass** das Stabilisierungselement (28), ausgehend vom ersten Haltebund (22) auf den weiteren Haltebund (26) hin in Umfangsrichtung gesehen sich verbreiternd ausgebildet ist.

14. Feuchte- und Wärmeaustauscher (10) gemäß einem der Ansprüche 9 bis 13, **dadurch gekennzeichnet, dass** der erste Haltebund (22) an seiner Außenumfangsfläche einen umlaufenden Haltewulst (23) aufweist.

15. Feuchte- und Wärmeaustauscher (10) gemäß einem der vorhergehenden Ansprüche, **dadurch gekennzeichnet, dass** er ein Sicherheitsventil (30) aufweist.

16. Feuchte- und Wärmeaustauscher (10) gemäß den Ansprüchen 15 und 16, **dadurch gekennzeichnet, dass** das Sicherheitsventil (30) mittels des Haltewulstes (23) des Haltebundes (22) gehalten ist.

17. Feuchte- und Wärmeaustauscher (10) gemäß einem der vorhergehenden Ansprüche, **dadurch gekennzeichnet, dass** der Filterkörper (16) eine zentrale Bohrung (17) aufweist.

## Claims

1. A moisture and heat exchanger (10), in particular for laryngectomy and/or tracheotomy patients, having
- a filter part (12), comprising a filter body (16) and a filter housing (18); and
- a tubular attachment part (14):
wherein
- the attachment part (14) is double-walled with an outer wall (32) and
an inner wall (34);
**characterised in that**:
- the distal end of the inner wall (34) is self-supporting and bears a distal end face (35); and
- the filter body (16) is at least partially held by a partial surface of the distal end face (35).

2. A moisture and heat exchanger (10) according to claim 1, **characterised in that** the distal end face (35) of the inner wall (34) of the attachment part (14) has at least one fixing means (42) for the filter body (16).

3. A moisture and heat exchanger (10) according to any one of the preceding claims, **characterised in that** the attachment part (14) is arranged directly adjacent to the filter part (12).

4. A moisture and heat exchanger (10) according to any one of the preceding claims, **characterised in that** the attachment part (14) and/or the filter part (12) has/have an annular cross-section.

5. A moisture and heat exchanger (10) according to any one of the preceding claims, **characterised in that** a circumferential bead (39) is arranged on an outer circumferential surface (38) of the attachment part (14), at the end of the latter remote from the filter part (12).

6. A moisture and heat exchanger (10) according to any one of claims 2 to 5, **characterised in that** the overall length of the inner wall (34) is shorter than the overall length of the outer wall (32).

7. A moisture and heat exchanger (10) according to any one of claims 2 to 6, **characterised in that** an annular cavity (44) is formed between the outer wall (32) and the inner wall (34) of the attachment part (14).

8. A moisture and heat exchanger (10) according to any one of the preceding claims, **characterised in that** the filter housing (18) has at least one lateral cut-out (20).

9. A moisture and heat exchanger (10) according to any one of the preceding claims, **characterised in that** the filter housing (12) has a first holding collar (22) held at a distance from the attachment part (14) by at least two crosspieces (24).

10. A moisture and heat exchanger (10) according to claim 9, **characterised in that** at its proximal end, the filter part (12) has another holding collar (26) which is connected to the attachment part (14).

11. A moisture and heat exchanger (10) according to claim 10, **characterised in that** the external circumference of the additional holding collar (26) is greater than that of the first holding collar (22).

12. A moisture and heat exchanger (10) according to any one of claims 9 to 11, **characterised in that** the crosspiece (24) has at least one stabilizing member (28).

13. A moisture and heat exchanger (10) according to claim 12, **characterised in that** viewed in the circumferential direction, the stabilizing member (28) widens, from the first holding collar (22) towards the additional holding collar (26).

14. A moisture and heat exchanger (10) according to any one of claims 9 to 13, **characterised in that** the first holding collar (22) has a circumferential holding bead (23) at its outer circumferential surface.

15. A moisture and heat exchanger (10) according to any one of the preceding claims, **characterised in that** it has a safety valve (30).

16. A moisture and heat exchanger (10) according to one of claims 14 and 15, **characterised in that** the safety valve (30) is held by means of the holding bead (23) of the holding collar (22).

17. A moisture and heat exchanger (10) according to any one of the preceding claims, **characterised in that** the filter body (16) has a central bore (17).

## Revendications

1. Échangeur de chaleur et d'humidité (10), en particulier pour sujet laryngectomisé et/ou trachéotomisé, comportant
- une partie filtrante (12), constituée d'un corps filtrant (16) et d'un boîtier (18) ; et
- une partie d'embase (14) de forme tubulaire ;
sachant que
- la partie d'embase (14) est réalisée à double paroi avec une paroi extérieure (32) et une paroi intérieure (34) ;
**caractérisé en ce que** :
- l'extrémité distale de la paroi intérieure (34) est réalisée de manière séparée et porte une face frontale (35) distale ; et
- le corps filtrant (16) est maintenu au moins en partie par une surface partielle de la face frontale (35) distale.

2. Échangeur de chaleur et d'humidité (10) selon la revendication 1, **caractérisé en ce que** la face frontale (35) distale de la paroi intérieure (34) de la partie d'embase (14) comporte au moins un moyen de fixation (42) pour le corps filtrant (16).

3. Échangeur de chaleur et d'humidité (10) selon l'une quelconque des revendications précédentes, **caractérisé en ce que** la partie d'embase (14) est disposée de manière directement adjacente à la partie filtrante (12).

4. Échangeur de chaleur et d'humidité (10) selon l'une quelconque des revendications précédentes, **caractérisé en ce que** la partie d'embase (14) et/ou la partie filtrante (12) ont une section annulaire.

5. Échangeur de chaleur et d'humidité (10) selon l'une quelconque des revendications précédentes, **caractérisé en ce qu'**un renflement (39) périphérique est agencé sur une face périphérique extérieure (38) de la partie d'embase (14) au niveau de l'extrémité de celle-ci, opposée à la partie filtrante (12).

6. Échangeur de chaleur et d'humidité (10) selon l'une quelconque des revendications 2 à 5, **caractérisé en ce que** la longueur d'encombrement de la paroi intérieure (34) est inférieure à la longueur d'encombrement de la paroi extérieure (32).

7. Échangeur de chaleur et d'humidité (10) selon l'une quelconque des revendications 2 à 6, **caractérisé en ce qu'**une cavité (44) annulaire est formée entre la paroi extérieure (32) et la paroi intérieure (34) de la partie d'embase (14).

8. Échangeur de chaleur et d'humidité (10) selon l'une quelconque des revendications précédentes, **caractérisé en ce que** le boîtier (18) du filtre comporte au moins un évidement (20) latéral.

9. Échangeur de chaleur et d'humidité (10) selon l'une quelconque des revendications précédentes, **caractérisé en ce que** le boîtier (18) du filtre comporte un premier collet de fixation (22), qui est maintenu à distance de la partie d'embase (14) par au moins deux nervures (24).

10. Échangeur de chaleur et d'humidité (10) selon la revendication 9, **caractérisé en ce que** la partie filtrante (12) comporte, au niveau de son extrémité proximale, un autre collet de fixation (26) relié à la partie d'embase (14).

11. Échangeur de chaleur et d'humidité (10) selon la revendication 10, **caractérisé en ce que** la périphérie extérieure du collet de fixation (26) supplémentaire est plus grande que celle du premier collet de fixation (22).

12. Échangeur de chaleur et d'humidité (10) selon l'une quelconque des revendications 9 à 11, **caractérisé en ce que** la nervure (24) comporte au moins un élément de stabilisation (28).

13. Échangeur de chaleur et d'humidité (10) selon la revendication 12, **caractérisé en ce que** l'élément de stabilisation (28), vu dans la direction circonférentielle, est réalisé en s'élargissant à partir du premier collet de fixation (22) vers le collet de fixation (26) supplémentaire.

14. Échangeur de chaleur et d'humidité (10) selon l'une quelconque des revendications 9 à 13, **caractérisé en ce que** le premier collet de fixation (22) comporte, sur sa face périphérique extérieure, un renflement de support (23) périphérique.

15. Échangeur de chaleur et d'humidité (10) selon l'une quelconque des revendications précédentes, **caractérisé en ce qu'**il comporte une soupape de sûreté (30).

16. Échangeur de chaleur et d'humidité (10) selon la revendication 14 ou 15, **caractérisé en ce que** la soupape de sûreté (30) est maintenue au moyen du renflement de support (23) du collet de fixation (22).

17. Échangeur de chaleur et d'humidité (10) selon l'une quelconque des revendications précédentes, **caractérisé en ce que** le corps filtrant (16) comporte une forure centrale (17).
